# DEMANDE DE BREVET EUROPEEN

(11) **EP 1 712 193 A1**
(43) Date de publication de la demande: **18.10.2006**
(21) Numéro de dépôt: 06356046.0
(22) Date de dépôt: 12.04.2006
(51) Int. Cl.: A61B 17/15, A61B 19/00, G06F 19/00, A61F 2/46

(54) **Dispositif chirurgical d'implantation d'une prothése partielle ou totale de genou**

(30) Priorité: 13.04.2005 FR 0503675
(71) Demandeur: TORNIER, 38330 Saint-Ismier (FR)
(72) Inventeur: Rochetin, Olivier, 42130 Marcilly Le Chatel (FR); Ratron, Yves-Alain, 38000 Grenoble (FR); Tornier, Alain, 38330 Saint Ismier (FR)
(74) Mandataire: Grand, Guillaume

(57) **Abrégé**

Ce dispositif chirurgical (1) comporte, d'une part, des moyens (2, 3, 4, 5, 7) de repérage dans l'espace de la rotule (R) et du fémur (F) d'un patient à opérer et, d'autre part, des moyens (2, 8) de représentation simultanée et relative de cette rotule, pourvue virtuellement d'un implant patellaire de la prothèse, et de ce fémur, pourvu virtuellement d'un implant fémoral de la prothèse, dans un plan transversal passant à la fois par la rotule et par la trochlée de l'implant fémoral précité. En utilisant ce dispositif, le chirurgien peut facilement et rapidement contrôler le positionnement congruent de l'implant patellaire vis-à-vis de la trochlée fémorale prothétique afin de réduire les risques de luxations ultérieures de la rotule prothésée.

## Description

La présente invention concerne un dispositif chirurgical d'implantation d'une prothèse partielle ou totale de genou.

Une prothèse totale de genou comporte de façon classique :
- une partie tibiale constituée généralement d'une embase métallique à solidariser fermement à l'extrémité supérieure réséquée du tibia et d'un patin en polyéthylène qui repose, de façon fixe ou mobile, sur la face supérieure de l'embase,
- un composant fémoral sous forme d'une coiffe généralement métallique qui, d'un côté, est fermement solidarisée à l'extrémité inférieure réséquée du fémur et qui, du côté opposé, délimite deux surfaces convexes reproduisant approximativement la géométrie de deux condyles fémoraux anatomiques interne et externe et destinées à reposer de manière articulée sur deux surfaces concaves correspondantes ménagées par le patin tibial, et
- un implant patellaire fermement solidarisé à la rotule anatomique, sur sa face postérieure, c'est-à-dire sa face tournée vers le fémur, après re-surfaçage de cette face.

La réussite de l'implantation d'une telle prothèse et sa tenue mécanique ultérieure en service sont grandement dépendantes du positionnement relatif des éléments prothétiques implantés. En pratique, on constate que les luxations de la rotule prothésée sont parmi les causes d'échecs les plus fréquentes. Les implants patellaires sont en effet traditionnellement posés sans grande considération des éléments fémoraux et tibiaux de la prothèse, le chirurgien se contentant généralement de centrer grossièrement l'implant patellaire sur la face postérieure réséquée de la rotule anatomique. Il est vrai que cet os est délicat à manipuler puisqu'il est porté par le tendon du quadriceps qu'il est nécessaire de retourner pour permettre le re-surfaçage de la rotule alors luxée.

US-A-2003/0212403 propose une méthode de pose d'une prothèse totale de genou, destinée à limiter l'invasion chirurgicale. A cet effet, ce document propose de repérer dans l'espace à la fois les os du tibia, du fémur et de la rotule et d'utiliser ces repérages pour guider les gestes du chirurgien sans que celui-ci n'ait à inciser complètement l'articulation du genou d'un patient. Le traumatisme pour le patient est donc amoindri et le travail du chirurgien est facilité. Cependant, le positionnement de l'implant patellaire est a priori décidé par le chirurgien sur la base des seules informations anatomiques relatives à la rotule à prothéser, sans prendre en considération les positionnements d'implantation des composants prothétiques tibial et fémoral. Les risques de luxation de la rotule ainsi prothésée sont donc analogues à ceux d'une prothèse implantée par une méthode conventionnelle plus invasive.

WO-A-02/36031 propose un système de détermination de la position d'une prothèse de genou à implanter, permettant à la fois de repérer dans l'espace les os du genou et d'afficher simultanément la rotule à prothéser et l'extrémité inférieure du fémur après que cette dernière ait été munie d'une prothèse fémorale. Aucune possibilité de représentation virtuelle d'implants n'est fournie par ce document, à la différence de WO-A-2004/001569 qui ne s'intéresse cependant pas à améliorer la mise en place d'un implant patellaire. Dans ces deux documents, les affichages proposés ne sont que suivant des vues en élévation des os du patient, notamment soit une vue correspondant à la face interne de la rotule, soit une vue axiale ou frontale de la zone inter-condylienne du fémur dans WO-A-02/36031.- De telles représentations en élévation ne sont pas suffisamment précises pour positionner efficacement les composants d'une prothèse de genou, en particulier pour limiter les risques de luxation d'une rotule prothésée.

Le but de la présente invention est de proposer un dispositif chirurgical qui assiste de manière précise, simple, rapide et économique le chirurgien lors de la chirurgie d'implantation d'une prothèse partielle ou totale de genou en vue de limiter le risque de luxations ultérieures de l'implant patellaire, en s'adaptant au mieux aux autres composants de la prothèse implantée, notamment à l'implant fémoral.

A cet effet, l'invention a pour objet un dispositif chirurgical d'implantation d'une prothèse partielle ou totale de genou comprenant au moins un implant patellaire et un implant fémoral, ledit dispositif comportant des moyens de repérage per-opératoire dans l'espace des os de la rotule et du fémur à prothéser, caractérisé en ce qu'il comporte des moyens de représentation per-opératoire adaptés pour, dans un plan transversal passant à la fois par la rotule et par une trochlée inter-condylienne de l'implant fémoral, représenter de façon simultanée et relative la rotule à prothéser pourvue virtuellement de l'implant patellaire et la partie inférieure du fémur à prothéser pourvue virtuellement de l'implant fémoral.

En utilisant le dispositif selon l'invention, le chirurgien peut, au cours de l'intervention chirurgicale, visualiser la représentation de l'implant patellaire virtuellement implanté sur la rotule d'un patient par rapport à la représentation de l'implant fémoral virtuellement implanté sur le fémur du patient et ainsi contrôler la congruence entre l'implant patellaire à implanter et la trochlée inter-condylienne de l'implant fémoral à implanter. Le chirurgien cherche en effet à obtenir la meilleure congruence possible en vue de limiter les risques de luxations ultérieures de la rotule vis-à-vis de l'implant fémoral, tant du côté interne qu'externe de la trochlée. Ce contrôle est particulièrement efficace dans le plan transversal, c'est-à-dire sensiblement perpendiculaire au tendon du quadriceps et aux tendons latéraux de l'articulation du genou, qui passe à la fois par l'os de la rotule et par la trochlée inter-condylienne de l'implant fémoral puisque les zones de transition externe et interne entre la trochlée et les condyles prothétiques sont alors bien visibles. Autrement dit, le fait que ce plan transversal passe par la matière osseuse constituant la rotule et la trochlée inter-condylienne garantit à la représentation des os et de leur implant virtuel une précision remarquable, ce qui permet au chirurgien d'estimer de manière fiable et rapide le centrage de l'implant patellaire vis-à-vis de la trochlée inter-condylienne, ainsi que les réglages appropriés pour implanter cet implant patellaire sur la rotule. Dans l'hypothèse où les moyens de représentation du dispositif chirurgical selon l'invention indiquent au chirurgien que la congruence escomptée est insuffisante, le chirurgien peut décider de gestes chirurgicaux permettant d'améliorer la configuration d'implantation, notamment en modifiant la géométrie d'implantation de l'implant patellaire et éventuellement celle de l'implant fémoral.

D'autres caractéristiques de ce dispositif, prises isolément ou selon toutes les combinaisons techniquement possibles, sont énoncées aux revendications dépendantes 2 à 10.

L'invention a également pour objet une méthode chirurgicale d'implantation d'une prothèse partielle ou totale de genou comprenant au moins un implant patellaire et un implant fémoral, dans laquelle, en per-opératoire, successivement :
- on repère dans l'espace les os de la rotule et du fémur d'un patient à traiter,
- on représente, de manière simultanée et relative, la rotule à prothéser pourvue virtuellement de l'implant patellaire et la partie inférieure du fémur à prothéser pourvue virtuellement de l'implant fémoral, cette représentation étant effectuée dans un plan transversal passant à la fois par la rotule et une trochlée inter-condylienne de l'implant fémoral,
- on règle l'implantation virtuelle de l'implant patellaire sur la rotule et éventuellement l'implantation virtuelle de l'implant fémoral sur le fémur de manière à ce que la partie postérieure de l'implant patellaire soit reçue de façon congruente dans la trochlée inter-condylienne fémorale,
- on sélectionne et on mémorise la configuration d'implantation de l'implant patellaire suivant le réglage obtenu, et
- on implante réellement l'implant patellaire sur la rotule du patient suivant la configuration d'implantation sélectionnée et mémorisée.

Suivant une caractéristique avantageuse de cette méthode :
- on compare, par rapport à la position de l'implant fémoral virtuellement implanté sur le fémur repéré dans l'espace, la position de l'implant patellaire virtuellement implanté sur la rotule repérée dans l'espace avec une position prédéterminée de cet implant patellaire dans laquelle les implants patellaire et fémoral sont positionnés de façon congruente, et
- l'implantation virtuelle de l'implant patellaire sur la rotule est réglée de manière à ce que sa position coïncide sensiblement avec la position prédéterminée.

L'invention sera mieux comprise à la lecture de la description qui va suivre, donnée uniquement à titre d'exemple et faite en se référant aux dessins sur lesquels :
- la figure 1 est une vue schématique d'une partie d'un dispositif chirurgical selon l'invention, appliqué au genou d'un patient à opérer ;
- la figure 2 est une vue en perspective d'une prothèse totale du genou à implanter au moyen du dispositif de la figure 1 ;
- la figure 3 est une vue en élévation selon la flèche III indiquée à la figure 2, d'une partie de la prothèse de genou dans un état de fonctionnement possible ;
- la figure 4 est une vue schématique de côté de l'articulation du genou du patient ;
- la figure 5 est une coupe selon un plan P de la figure 4 ;
- la figure 6 est une vue illustrant la représentation fournie par le dispositif de la figure 1, dans le plan P ;
- la figure 7 est une vue en coupe sagittale selon un plan S de la figure 2, du composant fémoral de la prothèse de la figure 2 ;
- la figure 8 est un schéma de la trajectoire de l'implant patellaire de la prothèse de la figure 2 par rapport à l'implant fémoral, au cours d'une sollicitation en flexion-extension de la prothèse de genou ; et
- la figure 9 est une vue illustrant une autre représentation fournie par le dispositif de la figure 1, dans le plan S.

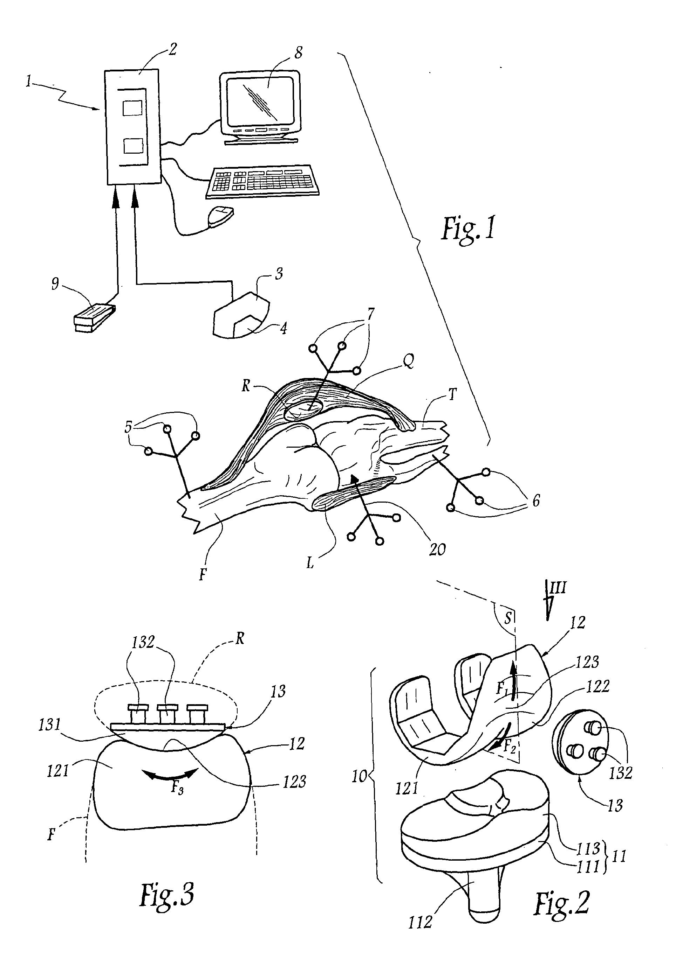

Le dispositif chirurgical 1 de la figure 1 comprend un ordinateur 2 associé à une unité d'émission et de réception de rayonnements infrarouges. Cette unité comporte un capteur 3 relié à l'ordinateur et une source d'alimentation infrarouge 4 couvrant le champ opératoire dans lequel est représenté en partie un genou d'un patient à traiter. Le genou comprend la partie inférieure d'un fémur F, la partie supérieure d'un tibia T, le tendon du quadriceps Q qui porte l'os de la rotule R et les tendons latéraux L qui relient le fémur et le tibia.

Pour permettre à l'ordinateur 2 de repérer dans l'espace les os du fémur F, du tibia T et de la rotule R, le dispositif 1 comporte des groupes respectifs de marqueurs 5, 6 et 7 qui renvoient, de façon passive, le rayonnement infrarouge en direction du capteur 3. Chaque groupe de marqueurs 5, 6 ou 7 forme un système de marquage tridimensionnel permettant à l'ensemble ordinateur 2-capteur 3 de suivre dans l'espace les déplacements respectifs du fémur, du tibia et de la rotule. L'utilisation de tels marqueurs est bien connue dans le domaine de l'orthopédie, par exemple par le document EP-A-1 249 213, et ils ne seront pas décrits ici plus avant.

Chaque groupe de marqueurs 5, 6 ou 7 est fixé à l'os du fémur, du tibia ou de la rotule au moyen d'une ou de plusieurs broches rigides. Ces broches sont positionnées sur les os de manière à laisser leurs marqueurs visibles en permanence pour le capteur 3.

L'ordinateur 2 du dispositif 1 est également associé à un ou plusieurs écrans 8 à même d'afficher des informations utiles au chirurgien, notamment les informations relatives à la position relative des os F, T et R et d'autres données décrites plus loin, de préférence sous forme de représentations graphiques comme détaillé ci-après.

Le dispositif 1 comporte également des moyens de commande 9, par exemple sous forme d'une pédale actionnable par le pied du chirurgien.

Le dispositif chirurgical 1 comporte en outre d'autres composants qui vont être détaillés ci-après lors de la description d'un exemple détaillé d'utilisation de ce dispositif en vue de l'implantation d'une prothèse totale de genou 10 représentée seule sur les figures 2 et 3. Cette prothèse est constituée d'une partie tibiale 11 à implanter dans l'os du tibia T, d'un composant fémoral 12 à implanter sur l'os du fémur F et d'un composant patellaire 13 à implanter sur l'os de la rotule R.

La partie tibiale 11 de la prothèse 10 comporte une embase rigide 111 globalement plane, munie, d'un côté, d'un pion 112 d'ancrage dans une extrémité préalablement réséquée du tibia T et équipée, du côté opposé, d'un plateau ou patin 113 relié à l'embase de manière fixe ou mobile selon le type de la prothèse 10. Sur sa face destinée à être tournée vers le composant fémoral 12, le plateau 113 définit deux surfaces concaves 113₁ et 113₂ conformées de manière à recevoir de façon articulée deux condyles interne 121 et externe 122 formés par l'implant fémoral 12 et reproduisant approximativement la géométrie de deux condyles fémoraux anatomiques. Les condyles 121 et 122 délimitent entre eux une trochlée fémorale 123 destinée à recevoir en appui l'implant patellaire 13, comme représenté à la figure 3. A cet effet, l'implant patellaire comporte un bouton 131 globalement en forme de calotte sphérique et trois pions 132 d'ancrage dans la partie postérieure de la rotule, portés en saillie vers l'avant par la face antérieure sensiblement plane du bouton 131, dont le centre est référencé O. La courbure concave de la trochlée fémorale 132 et la courbure convexe du bouton patellaire 131 sont dimensionnées pour que l'appui de l'implant patellaire 13 sur l'implant fémoral 12 soit congruent. Ce contact congruent permet ainsi, entre les implants 12 et 13, à la fois un mouvement relatif de translation le long de la trochlée fémorale 123, comme représenté par les flèches F₁ et F₂ à la figure 2, et des mouvements de pivotement relatifs autour des condyles 121 et 122, comme indiqué par la double flèche F3 à la figure 3.

La prothèse 10 décrite ci-dessus n'est donnée qu'à titre d'exemple et d'autres prothèses, de géométrie et/ou de nature différentes, peuvent être implantées au moyen du dispositif 1, suivant la méthode chirurgicale d'implantation décrite ci-après. En particulier, l'invention s'applique à la pose de prothèses partielles de genou, notamment aux prothèses fémoro-patellaires constituées d'un implant patellaire et d'un implant fémoral conçu pour s'appuyer directement sur l'extrémité supérieure du tibia anatomique et couramment appelé « implant trochléen ».

Dans un premier temps, le chirurgien incise le patient et recueille un certain nombre de données relatives à la géométrie anatomique des os du fémur F, du tibia T et de la rotule R du patient. A cet effet, différents moyens d'acquisition de ces données sont envisageables. A titre d'exemple, le chirurgien utilise un palpeur 20 repéré par l'ensemble ordinateur 2-capteur 3 et préalablement étalonné. Ce palpeur est amené sur des lieux remarquables des os à repérer et, à chacune de ces mises en place, le chirurgien actionne la pédale de commande 9 de manière à ce que l'ordinateur 2 enregistre la position du palpeur et, par là, en déduise les caractéristiques anatomiques du fémur F, du tibia T et de la rotule R. A partir de ces données, du suivi des marqueurs 5, 6 et 7 et de données pré-enregistrées relatives à une géométrie de base du genou, l'ordinateur 2 est capable de repérer dans l'espace les os F, T et R et de suivre leurs déplacements relatifs.

Durant cette étape d'acquisition de données, le tendon du quadriceps Q est retourné, comme représenté à la figure 1, de manière à ce que les marqueurs réfléchissants 7 soient visibles pour le capteur 3.

Dans un deuxième temps, le chirurgien indique à l'ordinateur 2 le modèle de la prothèse 10 qu'il compte implanter. Le choix de cette prothèse est soit laissé à l'entière discrétion du chirurgien, soit suggéré par l'ordinateur en tenant compte des caractéristiques morphologiques, en particulier osseuses, du patient opéré. Pour la suite de la description, on fait l'hypothèse que le chirurgien sélectionne la prothèse 10 des figures 2 et 3 comme celle qu'il souhaite implanter.

Dans un troisième temps, l'ordinateur 2 fournit au chirurgien, via l'écran 8, une représentation virtuelle des configurations d'implantation des composants fémoral 12 et patellaire 13 de la prothèse 10. Plus précisément, l'ordinateur représente sur son écran 8 la rotule R du patient pourvue virtuellement de l'implant patellaire 13 et la partie inférieure du fémur F pourvue virtuellement de l'implant fémoral 12. Pour permettre au chirurgien d'apprécier la bonne congruence entre le bouton prothétique 131 et la trochlée fémorale prothétique 123, cette représentation est effectuée dans un plan transversal P passant à la fois par la rotule R et la trochlée fémorale prothétique 123.

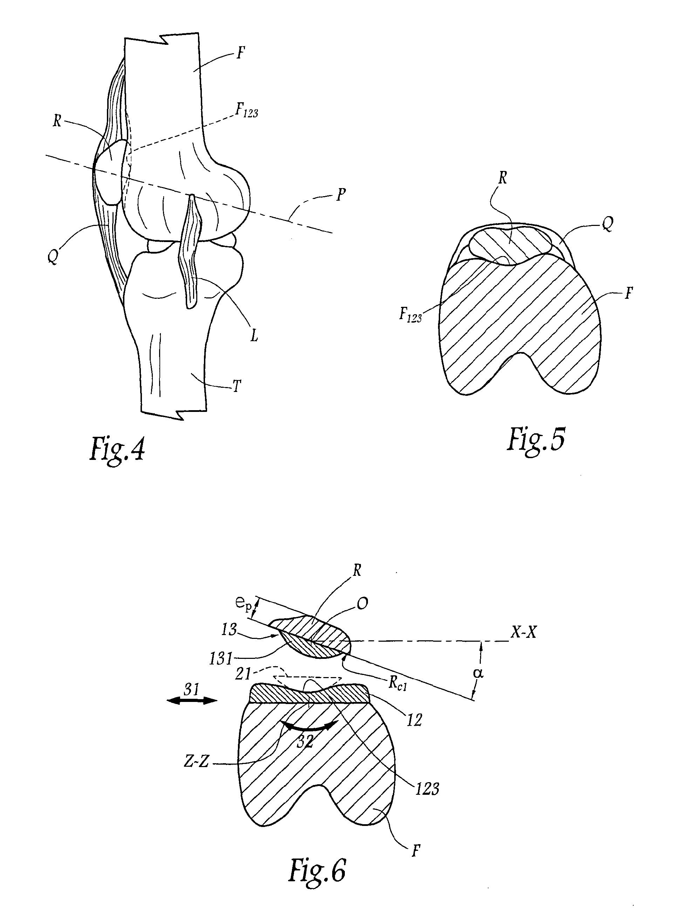

Pour bien comprendre l'intérêt de cette représentation, on a représenté sur la figure 4 l'articulation anatomique du genou de la figure 1, étant entendu que l'articulation représentée ne présente aucun désordre structurel notable. L'articulation est représentée en configuration d'extension si bien que la rotule R s'appuie sur la face antérieure de l'extrémité inférieure du fémur F, au niveau de la trochlée fémorale anatomique F₁₂₃. En coupant graphiquement cette articulation suivant le plan P indiquée à la figure 4, on observe à la figure 5 la congruence anatomique de la face postérieure de la rotule R vis-à-vis de la trochlée anatomique F₁₂₃. Ce plan P est transversal à la rotule, C'est-à-dire qu'il est sensiblement perpendiculaire au tendon du quadriceps Q et aux tendons latéraux L de l'articulation du genou, coupant ainsi la rotule au niveau de ses flans latéraux interne et externe. En représentant dans le plan P à la fois la rotule R pourvue virtuellement de l'implant patellaire 13 et le fémur F pourvu virtuellement de l'implant fémoral 12, le chirurgien dispose d'informations graphiques représentatives de l'implantation plus ou moins congruente des composants fémoral et patellaire de la prothèse 10. Un exemple de cette représentation est donné à la figure 6.

En pratique, le plan transversal P correspond avantageusement à un plan sensiblement médian de la rotule R, c'est-à-dire un plan transversal de part et d'autre duquel la matière osseuse constituant l'os de la rotule R se répartit de manière à peu près identique. Dans ce cas, le plan P correspond sensiblement à un plan de symétrie du bouton patellaire 131.

Ainsi, durant le troisième temps opératoire, le chirurgien contrôle si les implantations virtuelles des composants patellaire 13 et fémoral 12 placent de manière congruente le bouton patellaire 131 dans la trochlée fémorale prothétique 123. Si, comme à la figure 6, cette configuration congruente n'est pas obtenue, le chirurgien conclut que l'implantation de l'implant patellaire 13 sur la rotule R et/ou l'implantation de l'implant fémoral 12 sur le fémur F ne sont pas satisfaisantes et il les corrige. A cet effet, l'ordinateur 2 est équipé de moyens de réglage de ces implantations virtuelles, commandés par exemple de façon tactile sur l'écran 8. En particulier, le chirurgien peut alors régler, d'une part, la position de la ligne R_{c1} de coupe de la rotule dans le plan P, en particulier sa distance eₚ vis-à-vis du sommet de la face antérieure de la rotule et son angle α par rapport à une direction X-X medio-latérale fixe relative au patient, et d'autre part, la position de l'implant 13 le long de la ligne de coupe R_{c1}. Les moyens de réglage précités permettent également de changer la taille de l'implant patellaire 13 dans l'hypothèse où le chirurgien dispose d'un jeu de tels implants de tailles différentes renseignées dans l'ordinateur 2.

Vis-à-vis de l'implant fémoral 12, les moyens de réglage précités permettent avantageusement de modifier la taille de cet implant, sa position medio-latérale par rapport à l'os du fémur F, comme indiqué par la double flèche 31 à la figure 6, et/ou sa position angulaire par rapport au fémur autour d'une direction globalement verticale Z-Z préfixée, comme indiqué par la double flèche 32.

Pour faciliter le réglage des implantations virtuelles des composants prothétiques 12 et 13, la représentation de la figure 6 inclut avantageusement la trace en pointillés 21, dans le plan P, du bouton patellaire prothétique 131 dans une position prédéterminée par rapport à l'implant fémoral 12. Cette position prédéterminée, dont les caractéristiques géométriques sont par exemple fournies par le fabricant de la prothèse 10, est la position considérée comme optimale en vue de garantir la meilleure configuration congruente possible entre les composants patellaire et fémoral de la prothèse. Le chirurgien est aidé par cette trace puisqu'il peut alors modifier les configurations d'implantation virtuelle des implants 12 et 13 en vue de faire coïncider graphiquement le bouton rotulien 131 et la trace 21 précitée.

Avantageusement, le contrôle de la congruence entre les composants prothétiques 12 et 13 virtuellement implantés est effectué à la fois lorsque l'articulation de genou du patient est en extension, comme aux figures 4 et 5, mais également lorsque cette articulation est sollicitée en flexion. Ainsi, pour toute configuration fléchie du genou, sélectionnée par le chirurgien soit par une instruction informatique correspondante, soit par la sollicitation réelle de la jambe du patient opéré dont les mouvements sont suivis par les marqueurs 5, 6 et 7, l'ordinateur affiche une représentation analogue à celle de la figure 6, y compris, le cas échéant, une trace d'aide au chirurgien analogue à la trace 21, les données relatives aux différentes traces selon les différentes configurations de flexion-extension du genou étant fournies et mémorisées dans l'ordinateur 2. Les représentations successives obtenues sont effectuées dans le plan P fixe par rapport à la rotule R tandis que les vues en coupe du fémur F et de son implant virtuel 12 évoluent en fonction de la flexion-extension du genou, selon les déplacements relatifs entre la rotule et le fémur. Le réglage de l'implantation virtuelle des composants prothétiques 12 et 13 est ainsi possible quel que soit le degré de flexion de la jambe du patient. Le contrôle de la congruence est alors possible sur la course de flexion la plus critique du point de vue des risques de luxations ultérieures de la rotule, c'est-à-dire pour les degrés de flexion compris entre environ 0 et 30°.

En variante, le contrôle de congruence et le réglage de l'implantation virtuelle des composants prothétiques 12 et 13 ne sont effectués que pour une série discrète de configurations de flexion de l'articulation du genou, ces configurations étant choisies par le chirurgien comme les plus critiques du point de vue des risques de luxations de la rotule.

A la fin de ce troisième temps opératoire, le chirurgien fait mémoriser à l'ordinateur 2 les configurations d'implantation virtuelle dans le plan P des composants prothétiques 12 et 13 qu'il a réglées.

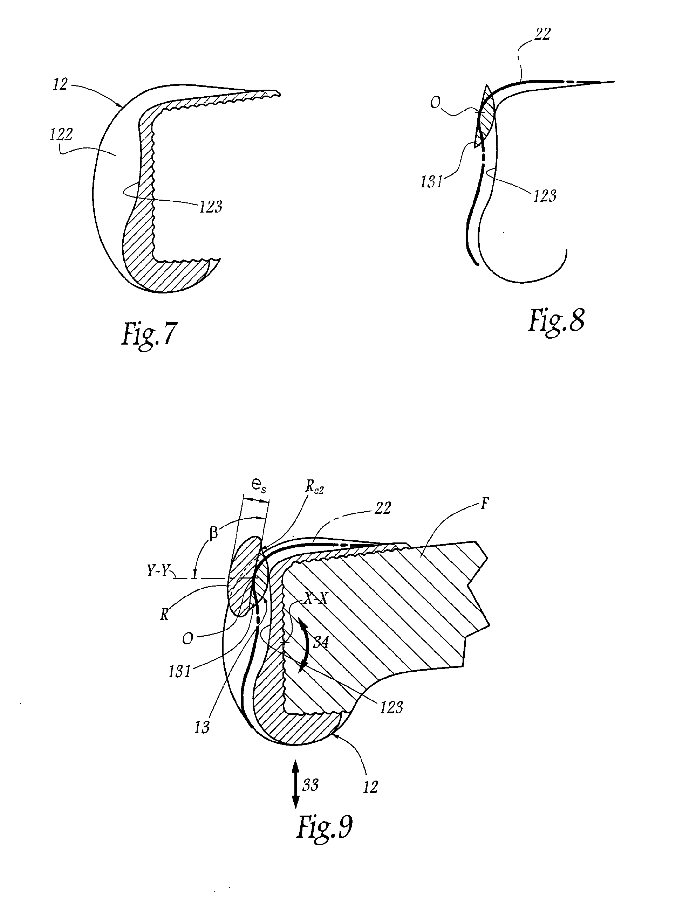

Dans un quatrième temps, le chirurgien contrôle que l'encombrement antéro-postérieur des composants fémoral 12 et patellaire 13 de la prothèse est sensiblement identique à l'encombrement correspondant anatomique du patient opéré afin que la prothèse, une fois implantée, ne perturbe pas de manière significative la tension musculaire et ligamentaire de l'articulation du patient. A cet effet, l'ordinateur 2 met à la disposition du chirurgien une représentation simultanée et relative de la rotule R pourvue virtuellement de l'implant patellaire 13 et de la partie inférieure du fémur F pourvue virtuellement de l'implant fémoral 12, et ce dans un plan sagittal passant à la fois par la rotule et par la trochlée fémorale prothétique 123, comme représenté sur la figure 9.

Pour bien comprendre l'intérêt de cette représentation dans le plan sagittal précité, on a représenté sur la figure 7 la coupe, dans ce plan, de l'implant fémoral 12. En pratique, le plan sagittal considéré correspond au plan S indiqué à la figure 2 passant par la ligne de fond de la trochlée 123. Sur la figure 8, on a représenté dans le plan S une ligne 22 qui correspond au parcours théorique du centre O du bouton patellaire 131 par rapport à l'implant fémoral 12 lorsque ce bouton est déplacé dans la trochlée prothétique 123 au cours d'un mouvement de flexion-extension de la prothèse de genou 10. Les données relatives à la ligne 22 sont par exemple fournies par le fabricant de la prothèse 10. Cette ligne est représentative d'une implantation optimale du composant patellaire 13 du point de vue de l'écartement antéro-postérieur entre ce composant 13 et le composant fémoral 12. Autrement dit, pour garantir que la composante antéro-postérieure du contact entre le bouton rotulien 131 de l'implant 13 ultérieurement posé et la trochlée fémorale 123 de l'implant 12 ultérieurement posé soit la plus proche possible de celle associée à la prothèse 10, les implantations des composants 12 et 13 doivent tendre, autant que possible, à placer sur la ligne 22 le centre O du bouton rotulien 131 effectivement implanté vis-à-vis du composant fémoral 12 effectivement implanté.

Ainsi, grâce à la représentation de la figure 9, le chirurgien peut contrôler, avantageusement au cours d'une sollicitation continue en flexion-extension de la jambe ou pour seulement quelques configurations de flexion de l'articulation du genou choisies par le chirurgien, que le centre O du bouton rotulien virtuel 131 est situé sur ou à proximité immédiate de la ligne théorique 22 décrite ci-dessus, dont les données caractéristiques ont été préalablement fournies à l'ordinateur 2. C'est par exemple le cas à la figure 9, où l'articulation du genou est représentée fléchie à environ 60°. L'implantation ultérieure des composants 12 et 13 permettra ainsi d'obtenir la cinématique prothétique escomptée, sans perturber de manière significative l'environnement musculaire et ligamentaire du genou opéré.

Dans l'hypothèse où le centre O est trop éloigné de la ligne 22, le chirurgien modifie la configuration d'implantation de l'implant patellaire virtuel 13 et/ou la configuration d'implantation de l'implant fémoral virtuel 12. En ce qui concerne l'implantation virtuelle patellaire, le chirurgien peut, en utilisant des moyens de réglage analogues à ceux décrits ci-dessus à propos de la figure 6 et portés par l'ordinateur 2 et l'écran 8, modifier la position de la ligne R_{c2} de coupe de la rotule dans le plan sagittal S, en particulier sa distance eₛ vis-à-vis du sommet de la face antérieure de la rotule et son angle β par rapport à une direction antéro-postérieure Y-Y préfixée, ainsi que la position de l'implant 13 le long de la ligne de coupe R_{c2}.

En variante, la trace de la ligne 22 n'est pas fournie au chirurgien sur l'écran 8, de sorte que le chirurgien ajuste les paramètres d'implantations sur la base du seul affichage des implants virtuels 12 et 13 par rapport aux os du fémur F et de la rotule R.

Dans l'hypothèse où un jeu de plusieurs implants patellaires 13 de tailles différentes est à disposition et où les données relatives à ce jeu ont été préalablement fournies à l'ordinateur 2, les moyens de réglage précités permettent également de changer la taille de l'implant virtuel 13 représenté par le dispositif 1.

En ce qui concerne l'implant fémoral 12, les moyens de réglage permettent avantageusement de modifier la taille de cet implant si un jeu de plusieurs implants fémoraux est prévu, sa position antéro-postérieure par rapport au fémur F, comme indiqué par la double flèche 33 à la figure 9, et/ou sa position angulaire par rapport au fémur autour de la direction médio-latérale X-X, comme indiqué par la double flèche 34.

A la fin de ce quatrième temps opératoire, le chirurgien fait mémoriser à l'ordinateur 2 les configurations d'implantation virtuelle dans le plan S des composants prothétiques 12 et 13 qu'il a réglées.

Dans un cinquième temps opératoire, le chirurgien résèque l'extrémité supérieure du tibia T, l'extrémité inférieure du fémur F et la partie postérieure de la rotule R. A cet effet, le chirurgien utilise des ancillaires de coupe repérés en permanence par l'ordinateur 2 de sorte que ce dernier guide les gestes du chirurgien en vue de réséquer les os précités selon les configurations d'implantation sélectionnées antérieurement, en particulier la configuration d'implantation rotulienne. Le document US-A-2003/0212403 décrit par exemple l'utilisation de guides de coupe tibiale, fémorale et rotulienne, aptes à être repérés par l'ordinateur 2.

On notera que le plan de coupe rotulienne est déduit par l'ordinateur 2 à partir des données relatives aux lignes de coupe R_{c1} et R_{c2}, réglées durant les troisième et quatrième temps opératoires.

Dans un sixième temps opératoire, le chirurgien implante les composants 11, 12 et 13 de la prothèse 10 au niveau des surfaces réséquées des os correspondants T, S et R.

Le dispositif 1 selon l'invention permet ainsi de positionner la prothèse 10 de façon optimale pour réduire autant que possible les risques de luxations ultérieures du composant patellaire 13. On notera que les six temps opératoires décrits ci-dessus sont effectués au cours d'une intervention chirurgicale proprement dite, c'est-à-dire durant laquelle le patient est par exemple sous anesthésie.

De plus, les différentes données enregistrées durant la pose de la prothèse peuvent être utilisées pour dresser un bilan post-opératoire et permettre ainsi de caractériser avec précision les capacités articulaires de la prothèse dans son état d'implantation. On remarquera cependant que les données acquises sont nettement moins nombreuses que celles nécessaires pour le fonctionnement d'un simulateur biomécanique du genou à opérer et que les moyens informatiques correspondants du dispositif selon l'invention sont donc moins onéreux et moins complexes à manipuler.

Divers aménagements et variantes au dispositif d'implantation 1 décrit ci-dessus sont en outre envisageables. A titre d'exemple, les moyens de repérage des os F, T et R ne sont pas limités à des marqueurs réfléchissant l'infrarouge ; des marqueurs sensibles aux ultrasons ou aux champs électromagnétiques sont par exemple utilisables.

## Revendications

1. Dispositif chirurgicàl (1) d'implantation d'une prothèse totale ou partielle de genou (10) comprenant au moins un implant patellaire (13) et un implant fémoral (12), ledit dispositif comportant des moyens (2, 3, 4, 5, 7) de repérage per-opératoire dans l'espace des os de la rotule (R) et du fémur (F) à prothéser, **caractérisé en ce qu'**il comporte des moyens (2, 8) de représentation per-opératoire adaptés pour, dans un plan transversal (P) passant à la fois par la rotule et par une trochlée inter-condylienne (123) de l'implant fémoral, représenter de façon simultanée et relative la rotule à prothéser (R) pourvue virtuellement de l'implant patellaire (13) et la partie inférieure du fémur à prothéser (F) pourvue virtuellement de l'implant fémoral (12).

2. Dispositif suivant la revendication 1, **caractérisé en ce que** le plan transversal (P) dans lequel la rotule (R) et le fémur (F) sont représentés par les moyens de représentation (2, 8) correspond à un plan sensiblement médian de la rotule.

3. Dispositif suivant l'une des revendications 1 ou 2, **caractérisé en ce qu'**il comporte une mémoire (2) de données relatives à un positionnement prédéterminé préférentiel de l'implant patellaire (13) par rapport à l'implant fémoral (12) pour toutes les configurations de flexion-extension du genou anatomiquement admissibles, et **en ce que** les moyens de représentation (2, 8) sont adaptés pour représenter, dans le plan transversal (P) et de manière simultanée et relative, à la fois la rotule (R) virtuellement prothésée, le fémur (F) virtuellement prothésé et une image d'aide au chirurgien (21) représentative d'au moins une partie (131) de l'implant patellaire (13), cette image d'aide étant positionnée par rapport à l'implant fémoral virtuel (12), selon la configuration de flexion-extension du genou considérée, en fonction des données de positionnement préférentiel contenues dans la mémoire.

4. Dispositif suivant l'une quelconque des revendications précédentes, **caractérisé en ce que** les moyens de représentation (2, 8) sont adaptés pour représenter la rotule (R) virtuellement prothésée, le fémur (F) virtuellement prothèse et, le cas échéant, l'image d'aide au chirurgien (21) pour différentes configurations de flexion-extension du genou, le plan transversal (P) dans lequel ces éléments sont représentés par ces moyens étant fixe par rapport à la rotule.

5. Dispositif suivant la revendication 4, **caractérisé en ce que** les moyens de représentation (2, 8) sont adaptés pour représenter lesdits éléments (R, F, 21) sur une course continue de flexion-extension du genou, notamment sur la course de flexion-extension maximale anatomiquement admissible.

6. Dispositif suivant l'une quelconque des revendications précédentes, **caractérisé en ce qu'**il comporte des moyens (2, 8) de réglage de l'implantation virtuelle de l'implant patellaire (13) sur la rotule (R).

7. Dispositif suivant la revendication 6, **caractérisé en ce que**, dans le cas où l'implant patellaire (13) est à poser au niveau d'une coupe postérieure de la rotule (R), les moyens de réglage (2, 8) sont adaptés pour modifier la distance (eₚ) entre un point prédéterminé de la face antérieure de la rotule (R) et la ligne (R_{c1}) de coupe rotulienne dans le plan transversal (P), la valeur d'angle (α) de la ligne de coupe rotulienne par rapport à une direction prédéterminée(X-X) et/ou la position de l'implant patellaire (13) le long de la ligne de coupe rotulienne.

8. Dispositif suivant l'une des revendications 6 ou 7, **caractérisé en ce que**, dans le cas où un jeu de plusieurs implants patellaires (13) de tailles respectives différentes est prévu, les moyens de réglage (2, 8) sont adaptés pour changer la taille de l'implant représenté.

9. Dispositif suivant l'une quelconque des revendications 6 à 8, **caractérisé en ce qu'**il comporte :
- des moyens (2) de sélection et de mémorisation d'au moins une configuration d'implantation de l'implant patellaire (13) réglée par les moyens de réglage (2, 8),
- un ancillaire de re-surfaçage de la face antérieure de la rotule (R), équipé de moyens de repérage dans l'espace, et
- des moyens de comparaison de l'action de resurfaçage imposée par cet ancillaire avec les données des moyens de sélection et de mémorisation.

10. Dispositif suivant l'une quelconque des revendications précédentes, **caractérisé en ce qu'**il comporte en outre d'autres moyens (2, 8) de représentation simultanée et relative de la rotule à prothéser (R) pourvue virtuellement de l'implant patellaire (13) et de la partie inférieure du fémur (F) à prothéser pourvue virtuellement de l'implant fémoral (12), dans un plan sagittal (S) passant à la fois par la rotule et la trochlée (123) de l'implant fémoral.
